# EUROPEAN PATENT SPECIFICATION

(11) **EP 2 084 290 B1**
(45) Date of publication and mention of the grant of the patent: **02.05.2012**
(21) Application number: 07818855.4
(22) Date of filing: 09.10.2007
(51) Int. Cl.: C12P 7/64, C12N 1/12, C12R 1/89, C12R 1/90

(54) **PRODUCTION OF OMEGA-3 FATTY ACIDS IN MICROFLORA OF THRAUSTOCHYTRIALES USING MODIFIED MEDIA**
HERSTELLUNG VON OMEGA-3-FETTSÄUREN IN DER MIKROFLORA VON THRAUSTOCHYTRIALES UNTER VERWENDUNG MODIFIZIERTER MEDIEN
PRODUCTION D'ACIDES GRAS OMÉGA-3 CHEZ UNE MICROFLORE DE THRAUSTOCHYTRIALES À L'AIDE DE MILIEUX MODIFIÉS

(30) Priority: 27.10.2006 EP 06022494; 27.10.2006 US 863138 P
(43) Date of publication of application: 05.08.2009
(73) Proprietor: Lonza AG, 4052 Basel (CH)
(72) Inventor: KIY, Thomas, 79539 Lörrach (DE); LUY, Markus, 3911 Ried-Brig (CH); ZEUMER, Oliver, 3912 Termen (CH)
(86) International application number: PCT/EP2007/008782
(87) International publication number: WO 2008/049512

(56) References cited:
- WO-A-94/08467
- WO-A-2005/035775
- WO-A-2005/045003
- WO-A-2005/045050
- US-B1- 6 410 281
- BAJPAI P K ET AL: "OPTIMIZATION OF PRODUCTION OF DOCOSAHEXAENOIC ACID (DNA) BY THRAUSTOCHYTRIUM AUREUM ATCC 34304" JOURNAL OF THE AMERICAN OIL CHEMISTS' SOCIETY, AOCS PRESS, CHAMPAIGN, IL, US, vol. 68, no. 7, July 1991 (1991-07), pages 509-514, XP008023474 ISSN: 0003-021X
- IIDA I ET AL: "Improvement of docosahexaenoic acid production in a culture of Thraustochytrium aureum by medium optimization" JOURNAL OF FERMENTATION AND BIOENGINEERING, SOCIETY OF FERMENTATION TECHNOLOGY, JP, vol. 81, no. 1, 1996, pages 76-78, XP002327281 ISSN: 0922-338X

## Description

The present invention relates to improved methods for the .production of omega-3 fatty acids by microorganisms belonging to Thraustochytriales using a modified composition of the culture medium. In particular, the invention is directed to a process for the production of omega-3 fatty acids by culturing microflora, such as strains of Ulkenia, Thraustochytrium and/or Schizochytrium, in fermentors which includes the step of culturing the Thraustochytriales microflora in an environment of reduced sodium ions and increased potassium ions.

The intake of certain omega-3 fatty acids has several beneficial effects on the human or animal body, including but not restricted to the reduction of cardiovascular and inflammatory diseases. It is known that several members of the order Thraustochytriales are excellent producers of polyenoic fatty acids in fermentors, especially when grown at low salinity levels, and thus can be used for the commercial production of omega-3 highly unsaturated fatty acids such as DHA, which is one of the most important omega-3 fatty acids.

Marine microalgae derived from the sea generally require defined conditions for growth and lipid production. For example the medium used for cultivation and/or fermentation of these organisms must have a certain salinity range in order to maintain growth and lipid production. From Bahnweg, Veröff. Inst. Meeresforsch. Bremerh., 17 (1979), 245-268, it is known that strains such as *Thraustochytrium aureum* grow best at NaCl concentrations in a range from 1.5% to 3% and that omission of potassium ions from the medium results in a reduced growth of these strains whereas growth of other strains such as *Schizochytrium aggregatum* seemed not to be affected. Furthermore, it is necessary that the medium must have a certain pH range. If this requirement is not fulfilled growth of the Thraustochytriales cells stops and/or the cells produce less polyenoic acids. According to Bahnweg, Veröff. Inst. Meeresforsch. Bremerh., 17 (1979), 245-268 the pH optimum for most of the Thraustochytriales strains is between pH 6.0 and pH 8.0 whereas no growth is observed below pH 5.0. However, during fermentation the pH changes with the consumption of nutrients in the fermentation broth and the release of waste products from the cells into the broth. US 5,340,594 describes that the culture medium becomes more alkaline during the fermentation and that thus the pH has to be controlled by acid addition. In contrast DE 103 52 837 A1 reports that during fermentation of marine microorganisms the culture medium acidifies if the pH is not controlled.

Very frequently media used for the fermentation of Thraustochytriales have a relatively high sodium chloride concentration. However, from US 6,410,281 B1 to Barcley it is known that such a high chloride concentration in medium used for the fermentation of Thraustochytriales promotes corrosion in stainless steel fermentors. Another problem encountered in the fermentation of Thraustochytriales is that the fermentation medium used although having a relatively high salinity level has a very low content of important metal ions, such as potassium, required for a high density cultivation or high lipid production of the Thraustochytriales.

The WO 2005/045003 discloses a process for cultivating microorganisms of the genus *Thraustochytriales,* according to which the microorganisms are cultivated in a low salt medium without adding sodium salts and chloride salts, the total salt content being less than 3.5 g/L (corresponding to less than 10 percent of sea water content), whereupon the PUFAs are isolated from the microorganisms and/or the medium.

The WO 2005/035775 discloses methods for the production of highly unsaturated fatty acids by marine microorganisms, including the heterotrophic marine dinoflagellate *Chryptecodinium,* using low levels of chloride ions. Specifically, methods of increasing the production of highly unsaturated fatty acids by marine microorganisms while growing in low chloride media by manipulating sodium ion and potassium ion levels are disclosed.

The US 6,410,281 discloses a process for growing the microflora Thraustochytrium, *Schizochytrium* and mixtures thereof, which includes the growing of the mircoflora in fermentation medium containng non-chloride containing sodium salts, in particular sodium sulphate.

The WO 2005/045050 discloses an optimized method for the production of PUFAs by cultivating microorganisms of the order *Thraustochytriales* in a fermentation medium that is pH-stabilized using calcium carbonate and comprises 3 to 15 g/L of CaCO₃, whereupon the PUFAs are isolated from the microorganisms and/or the medium.

Thus, there exists a need in the art for an improved medium for the fermentation of Thraustochytriales and also for an improved process for the cultivation of these microalgae enabling a higher density fermentation of Thraustochytriales cells in a fermentor and also enabeling the production of higher amounts of highly unsaturated fatty acids such as DHA therefrom while reducing negative side effects associated with the fermentation processs using a high salinity medium such as corrosion of the fermentor.

The present invention provides processes for the fermentation of microorganisms belonging to Thraustochytriales characterized in that during fermentation the pH of the medium is controlled by the addition of a base, in particular potassium hydroxide. Further, the present invention provides processes for obtaining lipids from microorganisms of Thraustochytriales characterized in that during fermentation of these microalgae the pH of the fermentation medium is controlled by the addition of a base, preferably potassium hydroxide.

Thus, according to the present invention, potassium hydroxide is used as pH controlling agent during the fermentation of Thraustochytriales strains. The addition of potassium hydroxide to the fermentation medium prevents that the pH of the medium is changed due to the consumption of certain nutrients and the formation of waste products by the microbial cells as the OH⁻ ions of the potassium hydroxide provide for the neutralisation of protons released into the medium. Also, by the addition of potassium hydroxide the concentration of potassium ions in the medium is remarkably increased which has a positive and advantageous effect on growth and lipid production of the cultivated microbial cells.

Furthermore, the use of potassium hydroxide as pH controlling agent in the inventive methods advantageously contributes to a considerably reduced corrosion of the equipment used for fermentation, in particular to a reduced corrosion of the stainless steel fermentor. In the prior art frequently acids are used as pH controlling agent. See for example US 5,340,594. One of the acids most frequently used in the art as pH controlling agent is HCl. Upon dissolution this acid dissociates in the negatively charged chloride ion and the positively charged hydrogen ion. However, anions such as the chloride ions remarkably contribute to corrosion of the stainless steel fermentor. See for example US 6,410,281. Therefore, in that patent it is suggested to use non-chloride sodium salts in the fermentation medium in order to reduce corrosion of stainless steel fermentors, e.g. sodium carbonate, sodium bicarbonate, sodium sulfate and the like. However, that patent did not pay attention to the fact that a considerable amount of ions contributing to corrosion of the fermentor is introduced by the addition of the pH controlling agent into the fermentation medium. In contrast to that patent, the present invention completely avoids the introduction of harmful anions by the pH controlling agent contributing to corrosion of the fermentor. Instead potassium hydroxide (KOH) used as pH controlling agent dissociates into potassium and hydroxide ions whereby the latter ions upon dissociation will be immediately neutralized by the hydrogen ions present in the fermentation medium. Thus, in comparison to US 6,410, 281 the present invention solves the problem of corrosion of fermentors in a substantially different way.

Thus, one aspect of the present invention relates to a process for the fermentation of microorganisms of the order Thraustochytriales comprising the step of cultivating the microorganisms under controlled conditions in a fermentation medium comprising a sodium concentration in the range of 0.2 g/liter to about 10.8 g/liter, adjusting the pH value of the medium during the fermentation with potassium hydroxide and harvesting the microalgae biomass.

Another aspect of the present invention relates to a method of reducing corrosion of a fermentor during the fermentation of Thraustochytriales microorganisms in a saline fermentation medium comprising cultivating the microorganisms in a fermentor under controlled conditions in a saline fermentation medium comprising a sodium concentration in the range of 0.2 g/liter to about 10.8 g/liter and adjusting the pH value of the medium during the fermentation with a base, in particular potassium hydroxide. The inventive process for reducing corrosion also includes the steps of harvesting the microbial biomass after fermentation and optionally the step of extracting lipids from the biomass.

The inventive process for fermentation of microorganisms and also the inventive process of reducing corrosion of a fermentor comprise the steps of cultivating and/or fermenting microalgae of Thraustochytriales such as Ulkenia, Thraustochytrium, Schizochytrium, Althornia, Aplannochytrium, Japanochytrium or Labyrinthuloides in a culture medium under defined conditions.

The liquid medium used for the processes according to the invention comprises a suitable carbon source such as a sugar. Preferred examples of a carbon source include, without being restricted to, glucose, starch, and molasses. The fermentation medium or fermentation broth used for the inventive processes also comprises a suitable nitrogen source such as a nitrate salt, ammonium compound, amino acids, yeast extract, corn steep liquor etc. The medium used for the inventive processes further comprises chloride ions at a concentration of about 0.2 g/liter to about 10.8 g/liter which corresponds approximately to the sodium ion concentration of sea water. Furthermore, the medium used for the cultivation and/or fermentation processes also contains potassium ions. In preferred embodiments of the invention the medium contains potassium ions in a concentration range of 0.1 g/liter to 1.0 g/liter.

Growth of the microalgae and lipid production by the inventive processes can be affected at any temperature allowing a satisfactory growth and/or a high lipid production of the cells, for example at a temperature between 15 °C and 48 °C, preferably at 25 °C to 28 °C. Growth of the microalgae can be carried out in vessels and tanks suitable for the fermentation or cultivation of microalgae. Preferred fermentation devices include, without being restricted to, stirred and/or aerated vessels, air lift reactors, shake flasks and the like. When cultivation of the Thraustochytriales microflora is carried out in a large vessel or reactor, it is preferred to produce an inoculum by inoculating a nutrient broth medium with an aliquot of a slant culture of one or more microalgae strains or an aliquot of a cryo-preserved culture of one or more strains. This inoculum is then transferred into liquid media of successively larger volumes until it has finally reached a volume suitable for inoculation into the final production volume.

In a preferred embodiment of the inventive process the Thraustochytriales microorganisms to be fermented belong to the genera Thraustochytrium, Schizochytrium, Ulkenia, Althornia, Aplananochytrium, Japanochytrium or Labyrinthuloides. Particular preferred examples of species or strains include, without being restricted to, *Ulkenia* SAM 2179, *Thraustochytrium aureum, Schizochytrium limacinum* SR21 and *Schizochytrium aggregatum.* The microalgae of the order Thraustochytriales belong to the group of heterotrophic organisms, i.e. they are organisms obtaining energy and cell carbon from organic substrates and being able to grow in the dark, i.e. in the absence of light.

According to the invention also mixtures of different species can be fermented. That means that two or more different Thraustochytriales species may be fermented simultaneously in the same fermentor in order to produce a mixed biomass comprising cells of at least two different species of Thraustochytriales and/or a mixed oil comprising lipids produced by two or more different species.

By cultivating two or more different species simultaneously in the same fermentor it is possible to obtain a biomass with a lipid profile that considerably differs from the lipid profile of one of these species alone. In a preferred embodiment of the invention *Ulkenia* SAM2179 and *Schizochytrium limacinum* SR21 are fermented together in order to produce a mixed biomass and also a mixed oil.

Usually the fermentation occurs at a temperature of more than 15 °C, preferably at a temperature of more than 20 °C, more preferred at a temperature of more than 25 °C such as about 28 °C. According to the present invention the microalgae are fermented or cultivated over a period of about 2 to about 14 days, preferably until the nutrient sources in the fermentation medium are exhausted.

According to the inventive process by the addition potassium hydroxide the pH value of the fermentation medium is maintained in a range of 3.5 to 8.0, preferably in a range of about 4.0 to about 7.0. Potassium hydroxide can be added to the culture medium either in solid form such as in form of potassium hydroxide pellets or in form of an aqueous solution.

According to the invention, the potassium hydroxide is added in such an amount to the medium that the final potassium ion concentration is at least about 0.2 g/liter, at least about 0.3 g/liter, at least about 0.4 g/liter, at least about 0.5 g/liter, at least about 0.6 g/liter, or at least about 0.7 g/liter.

It is preferred that the upper range of the potassium ion concentration is at most about 10 g/liter, at most about 6 g/liter, at most about 4 g/liter, at most about 3 g/liter, at most about 2.5 g/liter, at most about 2 g/liter, at most about 1.5 g/liter, or at most about 1 g/liter.

Most preferred concentrations of potassium ion are about 1.5 g/liter to about 3.0 g/liter, in particular 2.0 g/liter to 2.4 g/liter.

During fermentation, i.e. growth and propagation the Thraustochytriales cells accumulate lipids, in particular omega-3 highly unsaturated fatty acids. These cells containing lipids can be harvested in the exponential phase of growth or later, when the cells have reached their maximum cell density in order to obtain a biomass rich in omega-3 unsaturated fatty acids. If it is desired to obtain a biomass with a significantly increased omega-3 unsaturated fatty acid content, the culture of the thraustochytrids can be manipulated to become nutrient limited. In a preferred embodiment of the inventive process the culture is manipulated such that the nitrogen is limited for a suitable time, e.g. by transferring the culture to a nitrogen-free medium.

By the inventive processes a biomass density of the fermentation medium of more than 50 g/liter, preferably of more than 70 g/liter, more preferred of more than 90 g/liter and most preferred of more than 100 g/liter can be obtained. The dried biomass has a fatty acid lipid content of at least 20% by weight, preferably of at least 30% by weight, more preferably of at least 40% by weight and most preferably of at least 50% by weight. Generally, at least about 20% of the fatty acids or lipids produced by the Thraustochytriales microalgae in the inventive processes and contained in the biomass are polyunsaturated fatty acids, in particular omega-3 fatty acids such as DHA. Preferably, more than 30% of the lipids contained in the biomass are polyunsaturated fatty acids, in particular omega-3 fatty acids such as DHA. More preferred more than 40% or more than 50% of the fatty acids contained in the biomass are polyunsaturated fatty acids, in particular DHA.

Harvesting of the cells can be done for example by filtration techniques, centrifugation with or without previous complexation or flocculation by methods well-known in the art. For example the cells can be harvested by belt filtration, rotary drum filtration and the like. After harvesting the cells can be washed, frozen, lyophilized or spray-dried and stored under non-oxidizing conditions. After harvesting the obtained biomass rich in omega-3 unsaturated fatty acids, in particular rich in DHA, can also subjected to a drying process in order to obtain a dried biomass. Before drying optionally the harvested cell can be washed. Furthermore, a biomass obtained after fermenting a particular species of Thraustochytriales, can be mixed with a biomass obtained after fermenting of a second species of Thraustochytriales in order to obtain a biomass mixture. In a preferred embodiment an *Ulkenia* SAM2179 biomass is mixed with an biomass of *Schizochytrium limacinum.* The biomass obtained can be directly added to animal feed products or to food products destined for human consumption.

The present application thus also relates to a biomass or a biomass mixture obtained by the inventive processes and further the use thereof for the production of food and feed compositions.

Another aspect of the present invention relates to a process for obtaining lipids, in particular omega-3 unsaturated fatty acids such as DHA, from microorganisms belonging to Thraustochytriales comprising the step of cultivating the microorganisms under controlled conditions in a fermentation medium comprising a sodium concentration in the range of 0.2 g/liter to about 10.8 g/liter, adjusting the pH value of the medium during the fermentation with potassium hydroxide, harvesting the microalgae biomass and extracting lipids therefrom.

In this embodiment of the present invention the steps of cultivating the microorganisms under controlled conditions in a fermentation medium comprising a sodium concentration in the range of 0.2 g/liter to about 10.8 g/liter, adjusting the pH value of the medium during the fermentation with potassium hydroxide and harvesting the microalgae biomass can be done as outlined above. After harvesting the cells the thus obtained wet or dried biomass can be subjected to any one of extraction methods known in the art to be suitable for extracting lipids from microalgal cells. Extraction methods include, without being restricted to, supercritical CO₂ extraction, HPLC extraction, extraction by the use of solvents or mixtures of solvents such as hexane, chloroform, ether and methanol. Before such extraction methods are applied the cell walls of the cells of the harvested biomass can disrupted by techniques such as sonication, milling, freeze-thawing or enzymatic disrupture. The crude oil or crude lipids obtained by applying such an extraction method can be further purified by methods well-known in the arts, including, but not restricted to, refining, cold crystallization, etc. According to the invention it is possible to mix an oil obtained after fermentation of a given species of Thraustochytriales with a second oil obtained after fermentation of a second species of Thraustochytriales.

More than 20% of the thus obtained lipids are polyunsaturated fatty acids, in particular omega-3 fatty acids such as DHA. Preferably, more than 30% of the lipids thus obtained are polyunsaturated fatty acids, in particular omega-3 fatty acids such as DHA. More preferred more than 40% or more than 50% of the fatty acids thus obtained are polyunsaturated fatty acids, in particular DHA. The thus obtained lipids can be used for the production of food compositions, feed compositions, pharmaceutical compositions and cosmetic compositions.

Still another aspect of the present invention relates to a process for obtaining lipids, in particular omega-3 unsaturated fatty acids such as DHA, from microorganisms belonging to Thraustochytriales, said process comprising the step of cultivating the microorganisms under controlled conditions in a fermentation medium comprising a sodium concentration in the range of 0.2 g/liter to about 10.8 g/liter, adjusting the pH value of the medium during the fermentation with potassium hydroxide, and extracting lipids therefrom.

### Example 1

A inoculum culture was prepared according the following protocol.

A 2000 ml shake flask containing 350 ml of a sterile preculture medium consisting of Tropic Marin salt 16.7 g, glucose 30 g, yeast extract 10 g per 1000 ml distilled water, pH 6.0 was inoculated with 1 ml of cryo-preserved culture of *Ulkenia* SAM 2179. The shake flask was incubated over 68 hours at a temperature of 25 °C and a 160 rpm on a rotary shaker. After 68 hours the optical density at 660 nm was 16.1.

Then 135 ml (3%) of the thus prepared *Ulkenia* SAM 2179 inoculum culture were transferred to a fermentor containing a fermentation medium of the following composition: per 1000 ml tap water, dextrose 165 g, potassium phosphate 3 g, ammonium sulfate 5 g, magnesium chloride 1 g, sodium sulfate 1 g, calcium chloride 0.3 g, corn steep liquor 3.75 g, pH 4. The fermentor had a total volume of 5 liter and a working volume of 4.5 liter.

The fermentation was conducted at 28 °C and 380 rpm for approx. 166 hours. The aeration rate was 3.5 liter/min. For controlling the pH during fermentation a sterilized 20% KOH solution was prepared. The pH was automatically controlled such that it did not reach a value below 4.

After approx. 155 hours the main nutrients in the fermentation medium had depleted. At this time samples of the medium containing microbial cells were taken and the biomass quantity and lipid content were analyzed.

The thus taken samples of the broth rich in biomass were washed and then freeze-dried. Then the biomass samples were subjected to a transesterification with methanolic hydrochloric acid. The fatty acid profile and fatty acid content of the samples were determined by gas chromatography according to well-known standard protocols.

The following results were obtained: The dry weight of the biomass was 68.7 g/liter. The DHA content of this biomass was 16.1 g/liter. The DHA content of total fatty acids was 45%. The ratio between DHA and DPA, an omega-6 unsaturated fatty acid, was 3.5.

### Example 2

An inoculum culture was prepared according the following protocol.

A 2000 ml shake flask containing 350 ml of a sterile preculture medium consisting of Tropic Marin salt 16.7 g, glucose 30 g, yeast extract 10 g per 1000 ml distilled water, pH 6.0 was inoculated with 1 ml of cryopreserved culture of *Ulkenia* SAM 2179. The shake flask was incubated over 48 hours at a temperature of 25 °C and a 160 rpm on a rotary shaker. After 48 hours the optical density at 660 nm was 9.5.

Then 300 ml (3%) of the thus prepared *Ulkenia* SAM 2179 inoculum culture were transferred to a fermentor containing a fermentation medium of the following composition:

| | |
|---|---|
| Dextrose | 165 g/liter |
| corn steep liquor | 3.75 g/liter |
| KH₂PO₄ | 3.0 g/liter |
| NaCl | 0.8 g/liter |
| MgSO₄ × 7 H₂O | 1.5 g/liter |
| CaCl₂ × 2 H₂O | 0.3 g/liter |
| (NH₄)₂SO₄ | 5.0 g/liter |
| pH 4.0 | |

The fermentor had a total working volume of 10 liter.

The fermentation was conducted at 28 °C and 350 rpm for approx. 114 hours. The aeration rate was 7.5 liter/min. For controlling the pH during fermentation a sterilized 20% KOH solution was prepared. The pH was automatically controlled such that it did not reach a value below 4.

After approx. 110 hours the main nutrients in the fermentation medium had depleted. At this time samples of the medium containing microbial cells were taken and the biomass quantity and lipid content were analyzed.

The thus taken samples of the broth rich in biomass were washed and then freeze-dried. Then the biomass samples were subjected to a transesterification with methanolic hydrochloric acid. The fatty acid profile and fatty acid content of the samples were determined by gas chromatography according to well-known standard protocols.

The following results were obtained: The dry weight of the biomass was 62.3 g/liter. The DHA content of this biomass was 15.8 g/liter. The DHA content of total fatty acids was 44.3%. The ratio between DHA and DPA, an omega-6 unsaturated fatty acid, was 3.5.

## Claims

1. Process for the fermentation of microorganisms belonging to Thraustochytriales, **characterized in that** during fermentation of the microorganisms the pH value of the fermentation medium is controlled by the addition of potassium hydroxide, said process furthermore comprising the step of cultivating the microorganisms in a fermentation medium comprising a sodium concentration in the range of 0.2 g/liter to about 10.8 g/liter, adjusting the pH value of the medium during the fermentation with potassium hydroxide and harvesting the microalgae biomass.

2. Process for obtaining lipids from microorganisms belonging to Thraustochytriales, **characterized in that** during fermentation of the microorganisms the pH value of the fermentation medium is controlled by the addition of potassium hydroxide, said process furthermore comprising the step of cultivating the microorganisms in a fermentation medium comprising a sodium concentration in the range of 0.2 g/liter to about 10.8 g/liter, adjusting the pH value of the medium during the fermentation with potassium hydroxide, harvesting the microalgae biomass and extracting lipids therefrom.

3. Method for reducing corrosion of a fermentor during the fermentation of Thraustochytriales microorganisms, said method comprising cultivating the microorganisms in a fermentor in a saline fermentation medium comprising a sodium concentration in the range of 0.2 g/liter to about 10.8 g/liter and adjusting the pH value of the medium during fermentation by the addition of potassium hydroxide.

## Patentansprüche

1. Verfahren für die Fermentation von zu den Thraustochytriales gehörenden Mikroorganismen, **dadurch gekennzeichnet, dass** der pH-Wert des Fermentationsmediums während der Fermentation der Mikroorganismen durch Zugabe von Kaliumhydroxid kontrolliert wird, wobei das Verfahren weiterhin den Schritt des Kultivierens der Mikroorganismen in einem Fermentationsmedium mit einer Natriumkonzentration im Bereich von 0,2 g/Liter bis ungefähr 10,8 g/Liter, Einstellen des pH-Werts des Mediums während der Fermentation mit Kaliumhydroxid und Ernten der Mikroalgen-Biomasse umfasst.

2. Verfahren zum Gewinnen von Lipiden aus zu den Thraustochytriales gehörenden Mikroorganismen, **dadurch gekennzeichnet, dass** der pH-Wert des Fermentationsmediums während der Fermentation der Mikroorganismen durch Zugabe von Kaliumhydroxid kontrolliert wird, wobei das Verfahren weiterhin den Schritt des Kultivierens der Mikroorganismen in einem Fermentationsmedium mit einer Natriumkonzentration im Bereich von 0,2 g/Liter bis ungefähr 10,8 g/Liter, Einstellen des pH-Werts des Mediums während der Fermentation mit Kaliumhydroxid, Ernten der Mikroalgen-Biomasse und Extrahieren von Lipiden daraus umfasst.

3. Verfahren zum Reduzieren von Korrosion bei einem Fermenter während der Fermentation von Thraustochytriales-Mikroorganismen, wobei das Verfahren das Kultivieren der Mikroorganismen in einem Fermenter in einem salzhaltigen Fermentationsmedium mit einer Natriumkonzentration im Bereich von 0,2 g/Liter bis ungefähr 10,8 g/Liter und Einstellen des pH-Werts des Mediums während der Fermentation durch Zugabe von Kaliumhydroxid umfasst.

## Revendications

1. Procédé pour la fermentation de micro-organismes appartenant à Thraustochytriales, **caractérisé en ce que** pendant la fermentation des micro-organismes, la valeur du pH du milieu de fermentation est contrôlée par l'addition d'hydroxyde de potassium, ledit procédé comprenant en outre l'étape de culture des micro-organismes dans un milieu de fermentation comprenant une concentration en sodium dans la gamme de 0,2 g/litre à environ 10,8 g/litre, d'ajustement de la valeur du pH du milieu pendant la fermentation avec de l'hydroxyde de potassium et de récolte de la biomasse de micro-algues.

2. Procédé d'obtention de lipides à partir de micro-organismes appartenant à Thraustochytriales, **caractérisé en ce que** pendant la fermentation des micro-organismes, la valeur du pH du milieu de fermentation est contrôlée par l'addition d'hydroxyde de potassium, ledit procédé comprenant en outre l'étape de culture des micro-organismes dans un milieu de fermentation comprenant une concentration en sodium dans la gamme de 0,2 g/litre à environ 10,8 g/litre, d'ajustement de la valeur du pH du milieu pendant la fermentation avec de l'hydroxyde de potassium, de récolte de la biomasse de micro-algues et d'extraction de lipides de celle-ci.

3. Méthode pour réduire la corrosion d'un fermenteur pendant la fermentation de micro-organismes Thraustochytriales, ladite méthode comprenant la culture des micro-organismes dans un fermenteur dans un milieu de fermentation salin comprenant une concentration en sodium dans la gamme de 0,2 g/litre à environ 10,8 g/litre et l'ajustement de la valeur du pH du milieu pendant la fermentation par l'addition d'hydroxyde de potassium.
